# FASCICULE DE BREVET EUROPEEN

(11) **EP 2 590 593 B1**
(45) Date de publication et mention de la délivrance du brevet: **02.09.2020**
(21) Numéro de dépôt: 11741639.6
(22) Date de dépôt: 04.07.2011
(51) Int. Cl.: A61C 13/15

(54) **DISPOSITIF DE PHOTORÉTICULATION À BALAYAGE DE SPECTRE**
FOTOVERNETZUNGSVORRICHTUNG MIT SPEKTRALER ABTASTUNG
SPECTRAL SCANNING PHOTOCROSSLINKING DEVICE

(30) Priorité: 07.07.2010 FR 1055509
(43) Date de publication de la demande: 15.05.2013
(73) Titulaire: SOCIETE POUR LA CONCEPTION DES APPLICATIONS DES TECHNIQUES ELECTRONIQUES, 33700 Merignac (FR)
(72) Inventeur: NOUI, Hervé, F-11110 Salles D'aude (FR); LUCAS, Philippe, F-33127 Saint Jean D'illac (FR)
(74) Mandataire: Cabinet Beau de Loménie
(86) Numéro de dépôt international: PCT/FR2011/051569
(87) Numéro de publication internationale: WO 2012/004504

(56) Documents cités:
- EP-A2- 1 410 768
- WO-A1-2007/066112
- US-A1- 2001 046 652
- US-A1- 2003 091 955
- US-A1- 2008 268 401

## Description

### Arrière plan de l'invention

L'invention se rapporte à un dispositif ou appareil de photoréticulation apte à activer des matrices photoréticulables contenues dans des matériaux d'obturation, de reconstitution, d'empreinte, de collage, ou de blanchiment, d'application notamment dans le domaine dentaire, et comportant une source lumineuse, ainsi que des moyens optiques et électroniques pour contrôler, moduler, orienter, sélectionner et acheminer les énergies lumineuses vers une zone à éclairer.

La photoréticulation d'un matériau consiste en une réaction chimique, se produisant par exemple lors d'une polymérisation, qui lie entre elles de manière permanente les macromolécules constituant le matériau. Elle est provoquée par l'apport d'un rayonnement lumineux, permettant aux photo-amorceurs contenus dans le matériau d'engendrer des liaisons covalentes modifiant la structure du matériau, permettant ainsi d'obtenir les propriétés physiques recherchées telles que le durcissement du matériau ou son adhésion sur un support.

L'efficacité de la photoréticulation dépend en premier lieu du photo-amorceur et de son aptitude à créer des macro-radicaux, par exemple sur des chaînes polymères, il faut donc être en mesure d'irradier le matériau avec des longueurs d'onde comprises dans le spectre de photosensibilité du photo-amorceur présent dans le matériau. Or, les matériaux photoréticulables, utilisés par exemple dans le domaine dentaire, évoluent en permanence, de même que les photo-amorceurs entrant dans leur composition, ce qui conduit à une plus grande diversité des spectres de sensibilité à considérer.

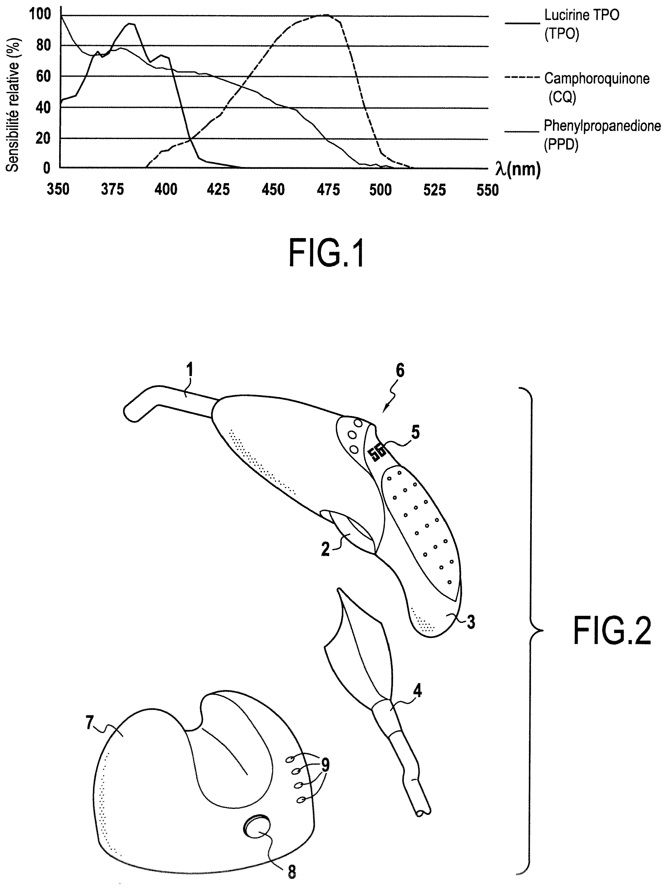

Afin de répondre aux besoins des praticiens, les dispositifs de photoréticulation doivent donc générer un large spectre lumineux apte à photoréticuler des matériaux contenant des photo-amorceurs variés tels que de la camphoroquinone (CQ) que du Phenylpropanedione (PPD) ou de la Lucirine dont les plages de longueur d'onde cibles correspondant au spectre de sensibilité de ces photo-amorceurs sont illustrées sur la figure 1.

De plus, cette réaction chimique est, pour la plupart des matériaux utilisés, une réaction exothermique engendrant une élévation de la température des zones exposées, élévation d'autant plus importante qu'elle s'ajoute à celle provoquée par l'absorption de l'énergie lumineuse par les tissus eux-mêmes.

Les dispositifs actuels, comme ceux disponibles pour le domaine dentaire, utilisent deux types de sources lumineuses, à savoir :
- Soit une source lumineuse qui émet un rayonnement lumineux distribué sur un large spectre, permettant de répondre au problème d'étendue spectrale
- Soit une source lumineuse qui produit un rayonnement sur spectre très étroit, voir monochromatique limitant l'énergie délivrée, et permettant ainsi de maitriser l'échauffement des tissus exposés

Pour ce qui concerne la première catégorie de source lumineuse, la source utilisée est mise en œuvre principalement avec des ampoules halogène, des lampes à arc (ex. xénon) ou à décharge.

Ce type de source lumineuse, produit un rayonnement lumineux de grande étendue spectrale. Cependant, au regard du rayonnement utile à la mise en œuvre des matériaux photoréticulables utilisés, le rendement de ces sources est assez faible tandis que leur coût d'achat et de maintenance reste élevé. En outre, ce type de source lumineuse requiert une importante quantité d'énergie pour son fonctionnement ainsi qu'un dispositif de refroidissement actif (convexion forcée) pour dissiper la chaleur émise. Il est, par conséquent, difficile de réaliser des dispositifs portables alimentés par des piles ou batteries rechargeables avec ce type de sources.

Par ailleurs, une partie non négligeable du rayonnement émis par ces sources à large spectre se trouve dans le domaine de l'infrarouge qui est susceptible de provoquer des effets thermiques indésirables sur les tissus à traiter (par exemple des nécroses), effets qui s'ajoutent à ceux de la réaction exothermique induite par la photoréticulation.

Enfin, un système complexe de filtre optique doit être mis en œuvre pour limiter la puissance radiométrique émise, afin de ne pas brûler les tissus traités par l'exposition aux rayonnements infrarouges.

La deuxième catégorie regroupe les dispositifs utilisant des diodes électroluminescentes (DEL ou LED). Ce type de source lumineuse présente l'intérêt de délivrer, dans la mesure où il correspond au matériau photoréticulable à traiter, un spectre de rendement optimal, puisque la totalité de l'énergie produite sera utile à la réaction chimique à engendrer. Les dispositifs utilisant ces sources présentent en outre une faible consommation en énergie et sont, par conséquent, aptes à être alimentés par une source d'énergie autonome constituée de piles ou de batteries rechargeables. Leur volume permet d'obtenir une ergonomie compacte. Enfin, les dispositifs optiques à mettre en œuvre avec ce type de sources peuvent être considérablement simplifiés car les diodes électroluminescentes sont généralement encapsulées dans des matériaux transparents ayant une transmission optimale. Ces matériaux sont par ailleurs moulés suivant une géométrie qui autorise la réalisation de dispositifs optiques intégrés permettant de collecter et diriger l'énergie lumineuse produite.

En raison de la faible plage de longueur d'onde couverte par le spectre d'émission d'une diode électroluminescente (de l'ordre de 20 nm pour une puissance de rayonnement ≥ 50%), les appareils équipés d'une seule diode ne peuvent pas fournir un spectre d'émission suffisamment large pour assurer la photoréticulation de plusieurs matériaux différents.

Pour tenter de remédier à cet inconvénient, il existe une solution qui consiste à augmenter la puissance énergétique délivrée à la diode électroluminescente au-delà de la valeur nominale habituelle. On augmente ainsi l'intensité du rayonnement émis par la diode aux marges de son spectre d'émission, ce qui permet d'élargir son spectre d'émission utile. Cependant, dans ce cas, on augmente l'intensité globale du rayonnement, ce qui entraîne des phénomènes d'élévation thermique sur les tissus qui sont difficilement supportables par les patients.

Par ailleurs, le praticien n'a pas toujours connaissance des photo-amorceurs contenus dans les matériaux qu'il utilise. Aussi, même s'il disposait d'une multiplicité d'appareils équipés de diodes électroluminescentes ayant des spectres d'émission différents, il serait néanmoins dans l'incapacité de savoir lequel utiliser.

Le document WO 2007 066112 divulgue une matrice de DEL et un procédé de pilotage d'une telle matrice. La matrice comprend une ou plusieurs paires DEL-capteur optique dans lesquelles le capteur optique mesure la lumière émise par la DEL associée. Dans une forme de réalisation de l'invention, une pluralité de jeux de DEL est disposée, chaque jeu émettant une lumière présentant une fréquence différente et chaque jeu étant pilotable pour produire une lumière émise désirée.

Le document EP 1 410 768 divulgue un instrument de polymérisation, comprenant une pluralité de sources lumineuses, dans laquelle chacune de la pluralité de sources lumineuses produit un faisceau lumineux incident ; et un moyen d'intégration de chacun des faisceaux lumineux incidents dans un faisceau lumineux de sortie, ledit faisceau lumineux de sortie ayant une distribution d'intensité de puissance de sortie, dans laquelle une première des sources lumineuses a une première longueur d'onde caractéristique et une deuxième des sources lumineuses a une deuxième longueur d'onde caractéristique.

Le document US 2001/0046652 divulgue des sources de lumière à diode électroluminescente pour le durcissement dentaire. Certains modes de réalisation comprennent des structures telles que des réseaux de diodes électroluminescentes, un dissipateur thermique, une dissipation thermique, un caloduc et des circuits de commande.

Le document US 2008/0268401 divulgue une unité de photopolymérisation, comprenant une LED ayant deux puces électroluminescentes ou plus pour émettre de la lumière dans différentes gammes de longueurs d'onde respectives parmi des gammes de longueurs d'onde pour le durcissement des photopolymères de sorte que la LED émet de la lumière dans une gamme de longueurs d'onde de 400 nm à 500 nm.

Le document US2003/0091955 divulgue un dispositif de durcissement à la lumière pour durcir une masse durcissable à la lumière appliquée à des fins dentaires qui possède une première diode électroluminescente pour émettre de la lumière à une gamme d'intensités correspondant à un ensemble de longueurs d'onde qui se chevauchent. Le dispositif de durcissement de la lumière comprend également une deuxième diode électroluminescente pour émettre de la lumière à une gamme d'intensités correspondant à un ensemble de longueurs d'onde qui se chevauchent.

### Obiet et description succincte de l'invention

L'invention a pour but de remédier aux inconvénients précités et de proposer un dispositif ou lampe de photoréticulation qui permet de couvrir, une plage de longueurs d'onde étendue apte à couvrir la longueur d'onde spectrale du photo-amorceur contenu dans le matériau, et ce tout en présentant une faible consommation énergétique au niveau du dispositif ne nécessitant pas l'utilisation de moyens de refroidissement actifs, et une émission d'énergie limitée permettant de contrôler l'échauffement des tissus exposés.

Ce but est atteint avec un dispositif de photoréticulation tel que défini dans la revendication 1, comprenant une source lumineuse destinée à initier la réticulation d'un matériau photoréticulable, dispositif dans lequel, ladite source lumineuse comprend au moins deux diodes électroluminescentes émettant chacune de la lumière dans une plage de longueur d'onde déterminée, lesdites plages de longueurs d'onde des diodes se chevauchant partiellement de manière à couvrir une plage de longueurs d'onde continue plus étendue que la plage de longueurs d'onde d'une seule diode, le dispositif de photoréticulation comprenant en outre des moyens d'activation pour activer sélectivement, c'est-à-dire l'une après l'autre, chaque diode électroluminescente.

Ainsi, en utilisant des diodes électroluminescentes ayant des spectres d'émission s'étendant sur des plages de longueurs d'onde différentes mais qui se chevauchent, il est possible de couvrir entièrement des plages longueurs d'onde étendues et d'assurer une initiation de la réticulation d'un grand nombre de photo-amorceurs, et ce sans avoir à connaître nécessairement la longueur d'onde cible.

En outre, l'activation sélective de chacune des diodes électroluminescentes dans l'appareil de l'invention permet de limiter la puissance de rayonnement émise et la consommation énergétique en comparaison avec, par exemple, des sources lumineuses utilisant des lampes halogènes, à arc ou à décharge, tout en assurant un balayage complet de la plage de longueurs d'onde étendue constituée par la réunion des spectres d'émission des diodes.

De tels avantages n'auraient pas pu être atteints avec une activation simultanée des diodes. En effet, dans un tel cas, l'appareil de photoréticulation doit être équipé d'un circuit électronique de puissance pour chaque diode et disposer d'une alimentation en énergie conséquente afin d'assurer l'alimentation simultanée des diodes ainsi que celle de moyens de refroidissement actif pour limiter l'émission de chaleur lors de l'activation simultanée.

Selon une caractéristique particulière de l'invention, le dispositif de photoréticulation comprend des moyens de commutation pour alimenter sélectivement chacune des diodes électroluminescentes à partir d'une source de courant unique.

Selon un autre aspect de l'invention, le dispositif de photoréticulation comprend des moyens de contrôle pour activer successivement au moins une fois chacune des diodes dans un intervalle de temps déterminé. Ainsi, on assure au moins un balayage complet de la plage de longueurs d'onde étendue constituée par la réunion des spectres d'émission des diodes, et ce même en cas d'arrêt anticipé d'un profil ou programme d'activation des diodes.

Selon un autre aspect ne faisant pas partie de l'invention, le dispositif de photoréticulation comprend des moyens de contrôle pour activer successivement plusieurs fois chaque diode et pour diminuer progressivement la puissance fournie aux diodes. On réduit ainsi le phénomène de sommation thermique ressentie par le patient et on limite les contraintes thermiques subies par le matériau à réticuler.

Selon encore un autre aspect de l'invention, le dispositif de photoréticulation comprend des moyens de contrôle pour activer successivement chaque diode sur une période d'activation déterminée, la diode émettant la chaleur de rayonnement la plus importante étant activée au début de la période d'activation et la diode émettant la chaleur de rayonnement la moins importante étant activée à la fin de la période d'activation. De même, un tel contrôle permet de mieux répartir les apports de chaleur au niveau de la zone à traiter et d'améliorer ainsi le confort du patient et de limiter les contraintes thermiques.

Selon une caractéristique particulière de l'invention, le dispositif comprend quatre diodes électroluminescentes ayant un spectre d'émission centré respectivement sur environ 420 nm, 440 nm, 460 nm et 480 nm de manière à couvrir une plage de longueur d'onde continue s'étendant entre au moins 410 nm et 490 nm, plage qui permet d'initier la réticulation de matériaux contenant des photo-amorceurs tels que de la camphoroquinone (CQ), du Phenylpropanedione (PPD) ou de la Lucirine.

Selon une autre caractéristique particulière de l'invention, le dispositif comprend en outre au moins un guide d'onde pour guider et/ou orienter le rayonnement lumineux émis par les diodes électroluminescentes vers une zone à traiter.

Selon un aspect particulier de l'invention, le dispositif comprend en outre des moyens de connexion aptes à recevoir une alimentation électrique à parti d'au moins une des sources d'alimentation suivantes: source d'alimentation autonome, secteur et bloc dentaire.

### Brève description des dessins

D'autres caractéristiques et avantages de l'invention ressortiront de la description suivante de modes particuliers de réalisation de l'invention, donnés à titre d'exemples non limitatifs, en référence aux dessins annexés, sur lesquels:
- la figure 1 est un graphique montrant des exemples de spectres de sensibilité pour différents photo-amorceurs,
- la figure 2 est une vue en perspective d'un appareil de photoréticulation conformément à un mode de réalisation de l'invention,
- la figure 3 est une vue éclatée en perspective montrant les éléments constitutifs d'un appareil de photoréticulation conformément à un mode de réalisation de l'invention,
- la figure 4 est une vue partielle en coupe suivant le repère AA de la figure 3,
- la figure 5 est un schéma fonctionnel d'un circuit électronique de commande d'un dispositif de photopolymérisation conformément à un mode de réalisation de la présente invention,
- la figure 6 est un graphique montrant une plage de longueurs d'onde étendue obtenue par la réunion des spectres d'émission de quatre diodes électroluminescentes,
- les figures 7 à 9 sont des exemples de profils ou programmes d'activation sélective des diodes électroluminescents qui peuvent être mis en œuvre avec l'appareil de photoréticulation de l'invention.

### Description détaillée des modes de réalisation de l'invention

La présente invention concerne un dispositif de photoréticulation destiné à appliquer sur un matériau photoréticulable un rayonnement de lumière dans une plage de longueurs d'onde donnée (spectres d'émission). On entend par matériau photoréticulable tout type de matériau dont la structure moléculaire se transforme sous l'effet d'un rayonnement lumineux d'une longueur d'onde donnée, en particulier par l'activation des photo-amorceurs contenus dans le matériau engendrant des liaisons covalentes modifiant la structure du matériau (durcissement, adhésion, etc.). A titre d'exemples non limitatifs, les matériaux photoréticulables peuvent être notamment des matériaux composites à durcir tels que des matériaux d'obturation, de reconstruction, d'empreinte, de collage ou des matériaux à activer tel qu'un produit de blanchiment. L'invention s'applique en particulier mais non exclusivement à des matériaux photoréticulables utilisés dans le domaine dentaire. L'invention s'applique également à des matériaux photoréticulables utilisés dans la formation de produits de revêtement de surface dans le domaine de la cosmétique ou autre.

Comme décrit plus loin en détail, le dispositif de photoréticulation comprend des moyens pour balayer une plage de longueur d'onde ou bande spectrale étendue correspondant à plusieurs plages de longueurs d'onde chacune émise respectivement par une diode électroluminescente déterminée, chaque diode correspondant à une longueur d'onde déterminée étant activée seule, c'est-à-dire avant ou après une autre diode correspondant à une autre longueur d'onde de la bande spectrale étendue.

La figure 2 illustre un dispositif de photoréticulation conformément à un mode de réalisation de l'invention et destiné à permettre la photoréticulation de matériaux d'empreinte et de reconstitution comme les composites notamment dans le domaine dentaire. Le dispositif est constitué d'une pièce à main 6 munie à son extrémité d'un guide optique stérilisable amovible 1. Ce dispositif est en outre muni d'un connecteur d'alimentation relié soit à une batterie rechargeable amovible 3, soit à une source d'alimentation externe correspondant à une alimentation réseau ou à celle d'un module intégrable en fauteuil dentaire, dont l'extrémité 4 vient s'enficher à la place de la batterie amovible 3. Une interface opérateur 5 permet de sélectionner et visualiser les profils ou programmes d'activation préenregistrés. L'activation se fait au moyen d'une gâchette de déclenchement 2. Un socle de recharge 7 permet de recharger la batterie 3. Ce socle de recharge comprend un moyen permettant de contrôler la puissance lumineuse émise, l'utilisateur introduisant l'extrémité du guide optique 1 dans la fenêtre d'entrée d'un capteur optique 8, le niveau de puissance lumineuse émise étant visualisé par exemple sur un écran d'affichage ou une échelle analogique à voyants lumineux 9.

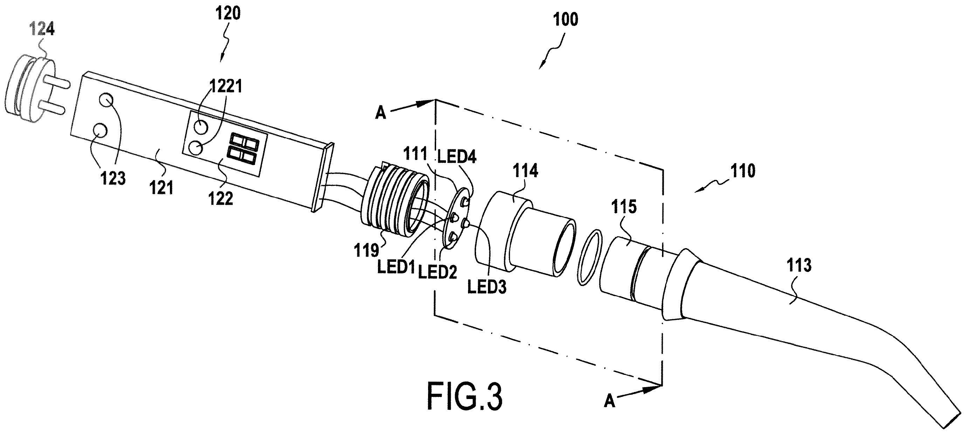

La figure 3 représente les éléments constitutifs d'un dispositif de photoréticulation 100, tel que celui de la figure 2 par exemple, conformément à un mode de réalisation de l'invention. Le dispositif de photoréticulation 100 comprend une partie antérieure 110 qui comprend une source lumineuse ou bloc optique 111 équipée de quatre diodes électroluminescentes LED1, LED2, LED3 et LED4 émettant chacune respectivement de la lumière dans une plage de longueur d'onde déterminée.

Les diodes électroluminescentes LED1 à LED4 sont couplées à un guide d'onde 113 permettant de guider, orienter et émettre l'énergie lumineuse produite par les diodes LED1 à LED4 en direction d'une zone d'éclairage correspondant au matériau photoréticulable à activer. Le guide d'onde 113 et le bloc optique 111 sont couplés au moyen d'un élément 114, le guide 113 étant monté de façon amovible à une extrémité de l'élément 114, le bloc optique 111 étant montée à l'autre extrémité de l'élément 114 sur un élément support 119.

Le guide d'onde 113 peut être constitué par des fibres optiques fusionnées, par un ensemble de lentilles, par un tube rigide de matériau optique, ou par une fibre optique liquide.

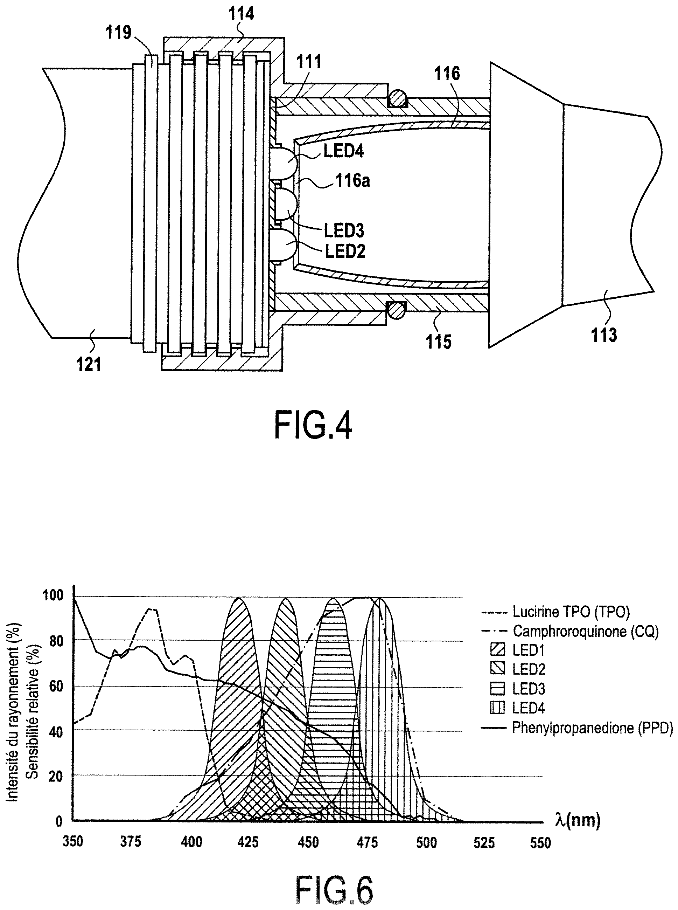

Le guide d'onde 113 est associé et guidé avec l'élément 114 au moyen d'un embout 115 qui, comme illustré sur la figure 4, comporte dans sa partie intérieure un réflecteur 116 permettant de réduire la divergence du rayonnement émis par les diodes LED1 à LED4 et comportant une ouverture centrale 116a pour loger ces dernières.

Le dispositif de photoréticulation 100 comprend une deuxième partie qui correspond à une unité de commande 120 située juste en dessous de la partie antérieure 110. Cette unité de commande 120 comprend une carte 121 équipée sur une face d'un écran 122 muni de témoins de fonctionnement et de sécurité 1221 et de touches de commande 123 et, sur l'autre face, d'un circuit électronique de commande (non représenté sur la figure 1). L'unité de commande est reliée, via des moyens de connexion 124, à une source d'alimentation en énergie électrique qui peut être notamment une source d'alimentation autonome constituée par des batteries rechargeables, une source d'alimentation externe telle que le secteur ou encore une source d'alimentation locale disponible par exemple sur le bloc dentaire du praticien. Les diodes LED1 à LED 4 du bloc optique 111 et le capteur d'intensité lumineuse 118 sont reliés électriquement au circuit électronique de commande.

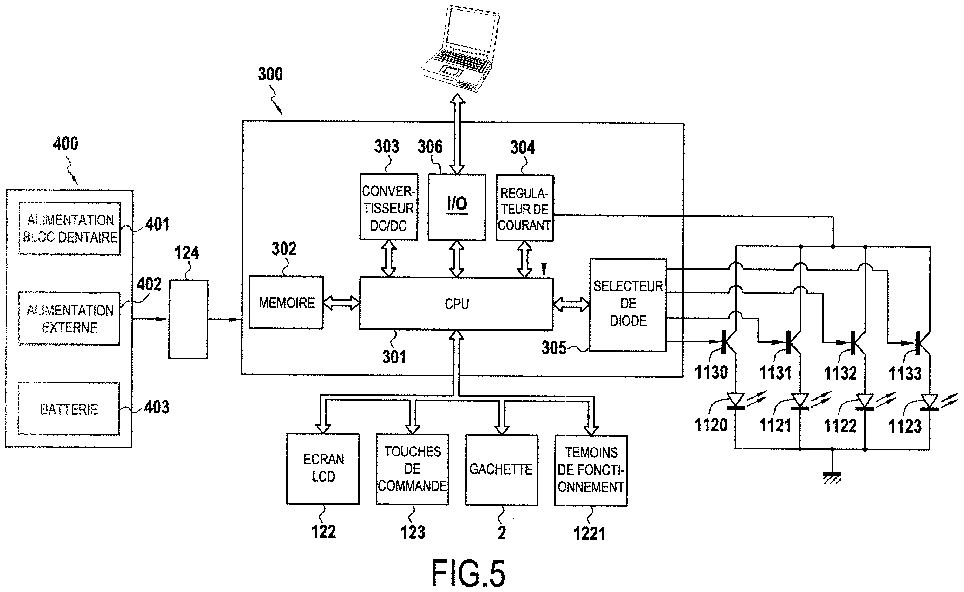

La figure 5 est un schéma fonctionnel d'un circuit électronique de commande 300 d'un mode de réalisation du dispositif de photoréticulation de l'invention, ce circuit étant disposé sur une face de la carte 121 de l'unité de commande 120 décrite ci-avant.

Le circuit 300 comprend une carte CPU 301 (par exemple un microcontrôleur programmable) qui est programmée pour piloter l'ensemble des paramètres de polymérisation. Cette carte comprend une mémoire non volatile 302 (par exemple une EEPROM) qui contient, sous forme de menus sélectionnables et éventuellement modifiables par une interface de téléchargement 306, les paramètres de photoréticulation à appliquer pour chaque menu. Des menus spécifiques conformes à l'invention seront détaillés plus loin.

Le praticien, au travers de l'écran LCD 122 et des touches de commande 123 sélectionne un des menus proposés puis déclenche le cycle de photoréticulation par la gâchette ou bouton de déclenchement 2 (cf. figure 2).

La carte CPU 301 pilote l'activation sélective des diodes électroluminescentes LED1 à LED4 du bloc optique 111 et contrôle la puissance délivrée à ces dernières. La carte CPU 301 paramètre et commande un convertisseur DC/DC 303 à découpage (PWM), ce qui permet de minimiser les élévations thermiques générées dans la pièce à main. Un régulateur de courant 304 asservit en permanence l'énergie envoyée aux diodes LED1 à LED4.

Concernant l'activation sélective des diodes permettant un balayage sur un large spectre conformément à l'invention, la carte CPU 301 contrôle l'activation de chaque diodes LED1 à LED4 via un sélecteur de diode 305 qui, en fonction des signaux de commande qu'il reçoit de la carte CPU active l'une ou l'autre de ces diodes. Dans le mode de réalisation décrit ici, l'activation sélective des diodes est mise en œuvre au moyen de transistors 1130 à 1133 reliés à la sortie du régulateur de courant 304 et dont l'état passant/non passant est contrôlé par le sélecteur 305.

Le circuit 300 est relié, via les moyens de connexion 124, à une source d'alimentation en énergie électrique 400 qui peut être indifféremment une source 401 provenant d'un bloc dentaire, une source d'alimentation externe 402 comme le secteur, ou une source d'alimentation autonome par batterie 403, comme par exemple des batteries de type Li-Ion, Ni-Cd, MnAl, etc., rechargeable par induction, contact ou autre.

Dans l'exemple décrit ici, les quatre diodes électroluminescentes LED1 à LED4 émettent chacune de la lumière dans un spectre d'émission ou plage de longueurs d'onde respectivement SpA, SpB, SpC et SpD comme illustré sur la figure 6. Plus précisément, le spectre d'émission de la diode LD1 est centré sur la longueur d'onde de 420 nm, le spectre d'émission de la diode LED2 est centré sur la longueur d'onde de 440 nm, le spectre d'émission de la diode LED3 est centré sur la longueur d'onde de 460 nm et le spectre d'émission de la diode LED4 est centré sur la longueur d'onde de 480 nm. On couvre ainsi une plage de longueur d'onde continue qui s'étend entre 400 nm et 500 nm (ou entre 410 nm et 490 nm pour intensité de rayonnement ≥ 50%) et qui recouvre au moins partiellement les spectres les longueurs d'onde cibles des photo-amorceurs tels que la camphoroquinone (CQ), le phenylpropanedione (PPD) et la lucirine.

Comme montré sur la figure 6, les spectres d'émission adjacents (ici SpA et SpB, SpB et SpC, et SpC et SpD) se chevauchent partiellement de manière à couvrir ensemble un spectre d'émission ou une plage de longueurs d'onde qui s'étend en continu depuis les premières longueurs d'onde couvertes par la diode 1120 jusqu'aux dernières longueurs d'onde couvertes par la diode 1123.

Les spectres d'émission se chevauchent de préférence à une longueur d'onde correspondant sensiblement à 50% de l'intensité du rayonnement émis par chaque diode électroluminescente. On assure ainsi une intensité de rayonnement minimale de 50% sur l'ensemble d'une plage de longueurs d'onde continue, qui s'étend ici alors entre 410 nm et 490 nm.

Conformément à l'invention, les diodes sont activées, c'est-à-dire alimentées en énergie, sélectivement les une après les autres. Cette activation sélective des diodes peut être réalisée suivant différents ordres (sélection des diodes) et selon des paramètres d'alimentation en énergie des diodes fixes ou variables.

Des exemples de profils ou programmes d'activation pour la photoréticulation sont maintenant décrits. Ces profils de photoréticulation peuvent être sélectionnés à partir de menus préenregistrés ou téléchargés dans la mémoire 302 du circuit électronique de commande 300. Ils correspondent chacun à une période de temps déterminée, appelés période d'activation, au cours de laquelle des séquences d'activation prédéfinies (périodiques ou non) des diodes sont mises en œuvre.

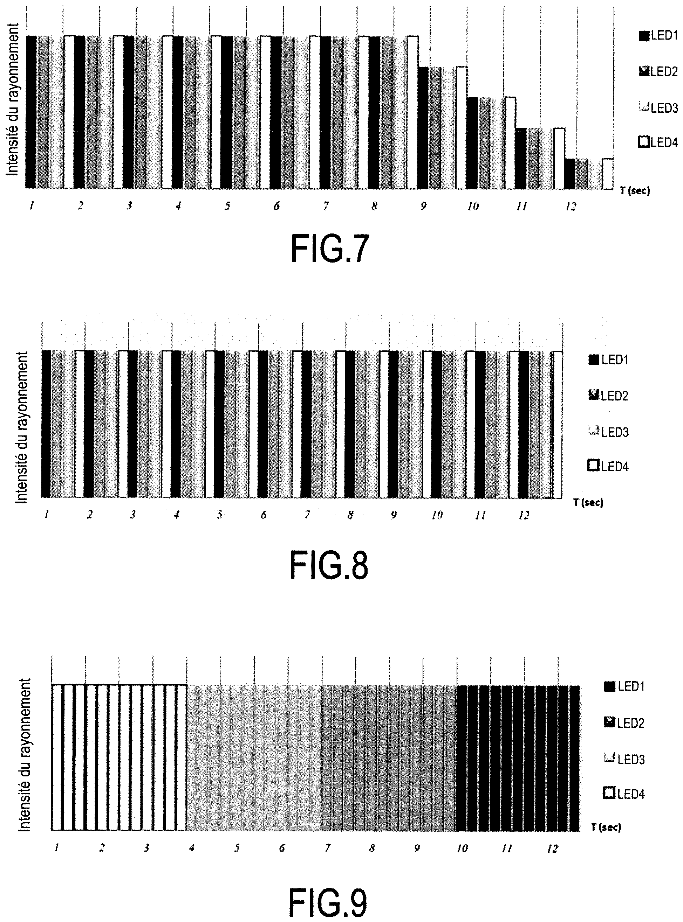

La figure 8 illustre un premier exemple de profil de photoréticulation dans lequel on répète au cours de la période d'activation plusieurs fois (ici 12 fois) la même séquence qui consiste à activer successivement chacune des diodes LD1 à LD4 à un niveau de puissance constant dans un intervalle de temps déterminé (ici 1 seconde). Avec un tel profil, la totalité la plage de longueur d'onde couverte par les spectres d'émission des quatre diodes est balayée à chaque séquence. Ainsi, on assure un balayage sur une plage de longueurs d'onde continue et étendue, et ce même en cas d'interruption après seulement une séquence (ici d'une durée d'une seconde).

La figure 7 illustre un deuxième exemple de profil de photoréticulation qui diffère du premier mode décrit ci-dessus en ce qu'on diminue progressivement la puissance délivrée aux diodes (diminution progressive du courant d'alimentation des diodes) sur les dernières séquences (ici les 4 dernières séquences). Dans ce profil d'activation, on réduit en fin de période d'activation la chaleur de rayonnement émise par les diodes, ce qui permet de limiter l'augmentation de chaleur en fin de cycle qui s'ajoute à la réaction exothermique de réticulation ou de polymérisation du matériau exposé. Dans le cas de la réticulation d'un matériau sur un patient, par exemple dans le domaine dentaire, la sensation d'augmentation excessive de chaleur ressentie par ce dernier peut être ainsi diminuée et son confort amélioré.

La figure 9 illustre un troisième exemple de profil de photoréticulation comprenant quatre séquences dans chacune desquelles une seule diode est activée, l'ordre des séquences étant déterminées de manière à activer, en début de période d'activation, les diodes émettant la chaleur de rayonnement la plus importante et, en fin de période d'activation, les diodes émettant la chaleur de rayonnement la moins importante. On évite ainsi une augmentation de chaleur trop importante en fin de cycle qui s'ajoute à la réaction exothermique de réticulation ou de polymérisation du matériau exposé. Le confort du patient est ainsi amélioré.

Le dispositif de photoréticulation n'est pas limité au mode de réalisation décrit ci-avant. En particulier, l'utilisation d'un guide d'onde n'est pas obligatoire, par exemple dans le cas où l'on utilise des diodes électroluminescentes encapsulées dans un module comprenant une lentille optique intégrée.

Par ailleurs, le dispositif de photoréticulation selon l'invention n'est pas limité à des appareils mobiles tels qu'une pièce à main dans le domaine dentaire. Il peut notamment avoir la forme d'un appareil de table formant une zone fixe d'exposition dans laquelle on place le matériau à photoréticuler, comme par exemple un produit de revêtement pour des ongles.

## Revendications

1. Dispositif de photoréticulation (100) comprenant une source lumineuse (111) destinée à initier la réticulation d'un matériau photoréticulable,
ladite source lumineuse (111) comprenant au moins deux diodes électroluminescentes (LED1-LED4) émettant chacune de la lumière dans une plage de longueur d'onde déterminée, lesdites plages de longueurs d'onde desdites au moins deux diodes se chevauchant partiellement de manière à couvrir une plage de longueurs d'onde continue plus étendue que la plage de longueurs d'onde de chacune des diodes,
le dispositif de photoréticulation comprenant en outre des moyens d'activation (300) pour activer sélectivement chaque diode électroluminescente,
le dispositif étant **caractérisé en ce qu'**il comprend des moyens de contrôle pour activer successivement chaque diode sur une période d'activation déterminée, la diode émettant la chaleur de rayonnement la plus importante étant activée au début de la période d'activation et la diode émettant la chaleur de rayonnement la moins importante étant activée à la fin de la période d'activation.

2. Dispositif selon la revendication 1, **caractérisé en ce qu'**il comprend des moyens de commutation (1130-1133) pour alimenter sélectivement chacune desdites diodes électroluminescentes (LED1-LED4) à partir d'une source de courant unique (400).

3. Dispositif selon la revendication 1 ou la revendication 2, **caractérisé en ce qu'**il comprend quatre diodes électroluminescentes (LED1-LED4) ayant un spectre d'émission centré respectivement sur environ 420 nm, 440 nm, 460 nm et 480 nm de manière à couvrir une plage de longueur d'onde continue s'étendant entre au moins 410 nm et 490 nm.

4. Dispositif selon l'une quelconque des revendications 1 à 3, **caractérisé en ce qu'**il comprend au moins un guide d'onde (113) pour guider et/ou orienter le rayonnement lumineux émis par les diodes électroluminescentes (LED1-LED4) vers une zone à traiter.

5. Dispositif selon l'une quelconque des revendications 1 à 4, **caractérisé en ce qu'**il comprend des moyens de connexion (124) aptes à recevoir une alimentation électrique à parti d'au moins une des sources d'alimentation suivantes: source d'alimentation autonome (403), secteur (402) et bloc dentaire (401).

## Patentansprüche

1. Fotovernetzungsvorrichtung (100), die eine Lichtquelle (111) umfasst, die dazu bestimmt ist, die Vernetzung eines fotovernetzbaren Materials einzuleiten,
wobei die Lichtquelle (111) mindestens zwei lichtemittierende Dioden (LED1-LED4) umfasst, die jeweils Licht in einem bestimmten Wellenlängenbereich emittieren, wobei die Wellenlängenbereiche der mindestens zwei Dioden sich teilweise derart überlappen, dass ein ununterbrochener Wellenlängenbereich abgedeckt wird, der ausgedehnter ist als der Wellenlängenbereich von jeder der Dioden,
wobei die Fotovernetzungsvorrichtung ferner Aktivierungsmittel (300) zum selektiven Aktivieren jeder lichtemittierenden Diode umfasst,
wobei die Vorrichtung **dadurch gekennzeichnet ist, dass** sie Steuerungsmittel zum aufeinanderfolgenden Aktivieren jeder Diode über eine bestimmte Aktivierungsperiode umfasst, wobei die Diode, welche die größte Strahlungswärme emittiert, am Beginn der Aktivierungsperiode aktiviert wird und die Diode, welche die kleinste Strahlungswärme emittiert, am Ende der Aktivierungsperiode aktiviert wird.

2. Vorrichtung nach Anspruch 1, **dadurch gekennzeichnet, dass** sie Umschaltmittel (1130-1133) zum selektiven Versorgen jeder lichtemittierenden Diode (LED1-LED4) mit Strom ausgehend von einer einzigen Stromquelle (400) umfasst.

3. Vorrichtung nach Anspruch 1 oder Anspruch 2, **dadurch gekennzeichnet, dass** sie vier lichtemittierende Dioden (LED1-LED4) umfasst, die ein Emissionsspektrum aufweisen, das auf ungefähr 420 nm, 440 nm, 460 nm bzw. 480 nm derart zentriert ist, dass ein ununterbrochener Wellenlängenbereich abgedeckt wird, der sich mindestens zwischen 410 nm und 490 nm erstreckt.

4. Vorrichtung nach einem der Ansprüche 1 bis 3, **dadurch gekennzeichnet, dass** sie mindestens einen Wellenleiter (113) zum Leiten und/oder Ausrichten der von den lichtemittierenden Dioden (LED1-LED4) emittierten Lichtstrahlung zu einer zu behandelnden Zone hin umfasst.

5. Vorrichtung nach einem der Ansprüche 1 bis 4, **dadurch gekennzeichnet, dass** sie Verbindungsmittel (124) umfasst, die geeignet sind, eine Stromversorgung ausgehend von mindestens einer von den folgenden Versorgungsquellen zu empfangen: unabhängige Versorgungsquelle (403), Stromnetz (402) und Dentaleinheit (401).

## Claims

1. A photo-curing device (100) comprising a light source (111) for initiating curing of a photo-curable material,
said light source (111) comprising at least two light-emitting diodes (LED1-LED4) each emitting light in a determined wavelength range, said wavelength ranges of said at least two LEDs overlapping partially so as to cover a continuous wavelength range that is broader than the wavelength range of each of the LEDs,
the photo-curing device further including activation means (300) for selectively activating each LED,
the device being **characterized in that** it includes control means for successively activating each LED over a determined activation period, the LED that emits the greatest radiation heat being activated at the beginning of the activation period and the LED that emits the least radiation heat being activated at the end of the activation period.

2. A device according to claim 1, **characterized in that** it includes switch means (1130-1133) for selectively powering each of said LED (LED1-LED4) from a single power supply (400).

3. A device according claim 1 or claim 2, **characterized in that** it has four LEDs (LED1-LED4) having respective emission spectra centered on about 420 nm, 440 nm,
460 nm, and 480 nm so as to cover a continuous wavelength range extending from at least 410 nm to 490 nm.

4. A device according to any one of claims 1 to 3, **characterized in that** it includes at least one waveguide (113) for guiding and/or aiming the light radiation emitted by the LEDs (LED1-LED4) towards a zone for treatment.

5. A device according to any one of claims 1 to 4, **characterized in that** it includes connector means (124) suitable for receiving electrical power from at least one of the following power supplies: a self-contained power supply (403), mains (402), and a dental unit (401).
